(19) Europäisches Patentamt
European Patent Office
Office européen des brevets

(11) **EP 3 598 127 A1**

(12) **EUROPEAN PATENT APPLICATION**

(43) Date of publication:
**22.01.2020 Bulletin 2020/04**

(51) Int Cl.:
***G01N 33/00*** *(2006.01)*

(21) Application number: **19187155.7**

(22) Date of filing: **18.07.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.07.2018 CN 201810795751**

(71) Applicant: **Honeywell International Inc.**
**Morris Plains, NJ New Jersey 07950 (US)**

(72) Inventors:
• **LIU, Ling**
**Morris Plains, NJ New Jersey 07950 (US)**
• **YAN, Xiaoyi**
**Morris Plains, NJ New Jersey 07950 (US)**

• **MU, Qinghui**
**Morris Plains, NJ New Jersey 07950 (US)**
• **WEI, Na**
**Morris Plains, NJ New Jersey 07950 (US)**
• **XIAO, Lei**
**Morris Plains, NJ New Jersey 07950 (US)**
• **ZHANG, Yang**
**Morris Plains, NJ New Jersey 07950 (US)**
• **YAO, Huiping**
**Morris Plains, NJ New Jersey 07950 (US)**
• **LIANG, Feng**
**Morris Plains, NJ New Jersey 07950 (US)**

(74) Representative: **Haseltine Lake Kempner LLP**
**Lincoln House, 5th Floor**
**300 High Holborn**
**London WC1V 7JH (GB)**

(54) **METHOD FOR DETECTING GAS CONCENTRATION AND GAS DETECTION DEVICE**

(57)     The present invention relates to a method for detecting a gas concentration using a gas detection device, comprising: determining a first relationship between a baseline drift of the gas detection device with respect to a gas concentration and an environmental temperature and performing, based on the baseline drift of the gas detection device with respect to a gas having a first temperature, a first compensation on a gas concentration output value of the gas having the first temperature determined by the gas detection device. With such a method, a gas concentration check result is more accurate, and the gas detection device can be applied to a wider range of application scenarios.

FIG. 1

EP 3 598 127 A1

## Description

TECHNICAL FIELD

The present invention relates to the field of gas detection technologies, and more specifically, to a method for detecting a gas concentration and a gas detection device.

BACKGROUND

When detecting gas concentrations or other indexes, the baseline is the benchmark for detection. When no gas is able to trigger detection device sensitivity, the baseline represents the impact of background noise or external interference on a detection device.

However, the baseline of the gas detection device may drift as the temperature of the external environment changes. In scenarios requiring high-precision detection, this issue cannot be ignored.

Furthermore, the sensitivity of the gas detection device to the detected gas may also vary according to the environmental temperature.

SUMMARY

The objective of the present invention is to provide a method for detecting a gas concentration, which can take into account the situation where a baseline of a detection device drifts due to environmental temperature changes.

In order to achieve the aforementioned objective, the present invention provides a technical solution as follows.

A method for detecting a gas concentration using a gas detection device, comprising: a) determining a first relationship between a baseline drift of the gas detection device with respect to a gas concentration and an environmental temperature; and b) performing, based on the baseline drift of the gas detection device with respect to a gas having a first temperature, a first compensation on a gas concentration output value of the gas having the first temperature determined by the gas detection device.

Preferably, the first relationship comprises a first functional relationship $Y=Fi(T)$, where T is the environmental temperature, and Y is the baseline drift.

Preferably, the first functional relationship is the following: $Y=a_1T^2+b_1T+C_1$, where $a_1$, $b_1$, and $c_1$ are constants determined via a fitting process.

Preferably, the first compensation is calculated according to the following formula: $C'=C-Y1$, where C is an original gas concentration output value of the gas detection device, Y1 is the baseline drift of the gas detection device with respect to the gas having the first temperature, and C' is a gas concentration output value of the gas detection device after the first compensation.

Preferably, the method further comprises: determining a second relationship between a sensitivity of the gas detection device to a gas and an environmental temperature; and separately calculating a first sensitivity of the gas detection device to a gas having a calibration point temperature and a second sensitivity to the gas having the first temperature according to the second relationship; and performing a second compensation on the second sensitivity based on the first sensitivity and an actual sensitivity of the gas detection device to the gas having the calibration point temperature.

Preferably, the second relationship comprises a second functional relationship $S=F_2(T)$, where T is the environmental temperature, and S is the sensitivity.

Preferably, the second functional relationship is the following: $S=a_2T^2+b_2T+C_2$, where $a_2$, $b_2$, and $c_2$ are constants determined via a fitting process.

Preferably, the second compensation is calculated according to the following formula: $S0'/S0= S1'/S1$, where S0 is the first sensitivity of the gas detection device to the gas having the calibration point temperature, S0' is the actual sensitivity of the gas detection device to the gas having the calibration point temperature, S1 is the second sensitivity of the gas detection device to the gas having the first temperature, and S1' is a sensitivity of the gas detection device to the gas having the first temperature after the second compensation.

Preferably, before the first compensation is performed, the second compensation is performed.

The present invention further provides a gas detection device, comprising a first compensation unit, configured to: acquire a baseline drift of the gas detection device with respect to a gas having a first temperature; and perform, based on the baseline drift, first compensation on a gas concentration output value of the gas having the first temperature determined by the gas detection device.

Preferably, the gas detection device further comprises a second compensation unit, configured to: acquire a first sensitivity of the gas detection device to a gas having a calibration point temperature and a second sensitivity to the gas having the first temperature; and perform a second compensation on the second sensitivity based on the first sensitivity and an actual sensitivity of the gas detection device to the gas having the calibration point temperature.

The present invention provides a method for detecting a gas concentration using a gas detection device, which takes into account a drift of a baseline of the gas detection device according to environmental temperature and performs compensation by means of experiments and fitting, so that a gas concentration check result of the gas detection device is more accurate, and the gas detection device can be applied to a wider range of application scenarios. In addition, such a method can be further combined with a sensitivity compensation step and is especially suitable for high-precision detection scenarios.

BRIEF DESCRIPTION OF THE DRAWINGS

FIG. 1 is a schematic flowchart of a method for detecting a gas concentration using a gas detection device provided in a first embodiment of the present invention.

FIG. 2 is a schematic flowchart of a method for detecting a gas concentration using a gas detection device provided in a second embodiment of the present invention.

FIG. 3 is a schematic modular structural diagram of a gas detection device provided in a third embodiment of the present invention.

DETAILED DESCRIPTION OF THE EMBODIMENTS

Specific details are provided in the following description to provide a thorough understanding of the present invention. However, those skilled in the art will clearly know that embodiments of the present invention can be implemented without these specific details. In the present invention, specific numeric references such as "first element" and "second device" may be made. However, the specific numeric reference should not be construed as a literal sequential order but rather be construed that the "first element" is different from a "second element".

The specific details provided in the present invention are only exemplary. The specific details may be varied and still fall within the spirit and scope of the present invention. The term "coupling" is defined to represent a direct connection to a component or an indirect connection to a component via another component.

Preferred embodiments suitable for implementing methods, systems, and devices of the present invention are described below with reference to the accompanying drawings. Although each embodiment is described with respect to a single combination of elements, it should be understood that the present invention includes all possible combinations of the disclosed elements. Thus, if one embodiment includes elements A, B, and C, while the second embodiment includes elements B and D, the present invention should also be considered to include other remaining combinations of A, B, C or D, even if not explicitly disclosed.

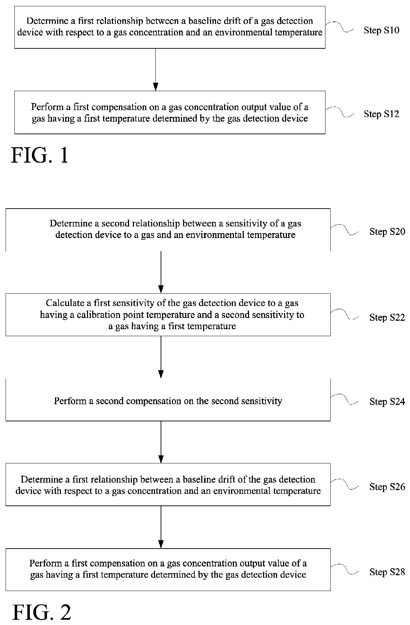

As shown in FIG. 1, a first embodiment of the present invention includes steps S10 and S11.

Step S10: determine a first relationship between a baseline drift of a gas detection device with respect to a gas concentration and an environmental temperature.

For example, the first relationship is expressed as $Y=F_1(T)$, where Y is the baseline drift, and T is the environmental temperature. Statistics of experimental data show that the first relationship is in close proximity with a quadratic functional relationship, or is a parabolic function.

As an example, the baseline drift Y and the environmental temperature T satisfy a first equation: $Y=a_1T^2+b_1T+C_1$, where $a_1$, $b_1$, and $c_1$ are constants determined via a (first) fitting process. The first fitting process may be performed by factory technicians when the gas detection device is delivered from the factory, or may be performed by technicians at intervals after application.

In the first equation, T is the temperature in the unit of degrees Celsius, Y represents the baseline drift value, and the unit thereof is the same as that of the gas concentration, namely, PPM.

In the fitting, the environmental temperature and corresponding baseline drifts are first respectively measured through experiments for a plurality of groups of gases to be detected and are recorded in a relationship table. Then, the environmental temperature and corresponding baseline drifts are substituted as T values and Y values into the fitting equation for parameter solution. For example, a least squares method or a linear recursive algorithm may be adopted as the fitting algorithm. The specific values of $a_1$, $b_1$, and $c_1$ can be determined after fitting.

It should be noted that since the gas is stored in the environment, in most cases the temperature of the gas passing through the gas detection device is similar to the temperature of the environment. However, the present invention is also applicable to scenarios where the gas temperature is different from the environmental temperature.

Step S11: perform, based on the baseline drift of the gas detection device with respect to a gas having a first temperature, first compensation on a gas concentration output value of the gas having the first temperature determined by the gas detection device.

In order to counteract the influence of the environmental temperature on the baseline of the gas detection device, in the present invention, the baseline drift value obtained by fitting is subtracted from the gas concentration output value of the gas having the first temperature determined by the gas detection device. The baseline drift value may be a positive or negative value.

The first compensation may be performed in the following manner: calculating C'=C-Y1, where C is the gas concentration output value of the gas having the first temperature originally measured by the gas detection device, and Y1 is the baseline drift of the gas detection device with respect to the gas having the first temperature, the specific value of which can be directly determined according to the first equation (into which the first temperature is substituted). C' is a gas concentration output value of the gas detection device after the first compensation.

Compared with the original value C, C' reflects the situation where the baseline drifts with the environmental temperature, so that a detection result of the gas detection device is more precise; meanwhile, the adaptability of the gas detection device to different application scenarios is expanded.

It can be understood that in the case that the gas detection device is used for measuring concentrations of a plurality of gases, a fitting process is performed once for each gas so as to obtain a fitting equation corresponding to the gas type. When a specific gas is detected, a concentration output value of the gas is correspondingly compensated according to a fitting equation corresponding to the gas.

As shown in FIG. 2, a second embodiment of the present invention includes steps S20, S22, S24, S26, and S28.

It should be noted that although the second embodiment shows that steps S26 and S28 are performed after steps S20 to S24, it should be understood that the second embodiment is merely a preferred embodiment of the present invention. In fact, steps S26 and S28 may also be performed first, and then steps S20 to S24 are performed.

In addition, after reading through the specification of the present invention, those skilled in the art may make simple variations, combinations or omissions of the steps or otherwise recombine the steps, all of which should fall within the scope of the present invention. For example, step S22 may be incorporated into and performed together with step S20 or step S24 without being listed separately.

Step S20: determine a second relationship between a sensitivity of the gas detection device to a gas and an environmental temperature.

The second relationship may be expressed as a second functional relationship $S=F_2(T)$, where T is the environmental temperature, and S is the sensitivity. The second functional relationship is more likely to exhibit as a quadratic functional relationship. As an example, the sensitivity S of the gas detection device to the gas and the environmental temperature T satisfy a second equation: $S=a_2T^2+b_2T+C_2$, where $a_2$, $b_2$, and $c_2$ are constants determined in a (second) fitting process. The second fitting process may be performed by a manufacturer of the gas detection device, or may be performed by technicians in the specific application environment before applying the device to detect the gas concentration.

Step S22: separately calculate a first sensitivity of the gas detection device to a gas having a calibration point temperature and a second sensitivity to a gas having a first temperature according to the second relationship.

In this step, the calibration point temperature and the first temperature are separately substituted into the second equation obtained by fitting according to step S20, so that the corresponding first sensitivity (corresponding to the calibration point temperature) and second sensitivity (corresponding to the first temperature) can be directly obtained. It should be understood that the calibration point temperature may be changed, and may be initially set to an environmental temperature in a specific application scenario or a common temperature of a gas to be detected. Thereafter, the calibration point temperature may be reset according to each interval as required.

Step S24: perform a second compensation on the second sensitivity.

Specifically, in this step, the second compensation is performed on the second sensitivity based on the first sensitivity and an actual sensitivity of the gas detection device to the gas having the calibration point temperature; with the compensation, the sensitivity of the gas detection device to the gas can counteract the effects of environmental temperature changes, so as to improve the precision and adaptability of the gas detection device.

It is determined through experiments that the compensated sensitivity and the original sensitivity have a linear or similar linear relationship. The ratio between them is close to the ratio between the actual sensitivity of the gas detection device when the gas having the calibration point temperature is provided and the calculated sensitivity (calculated according to the second equation).

Thus, as an example, the second compensation is calculated according to the following formula: $S0'/S0=S1'/S1$, where S0 is the first sensitivity of the gas detection device to the gas having the calibration point temperature and is obtained in step S22, S0' is the actual sensitivity of the gas detection device to the gas having the calibration point temperature, S1 is the second sensitivity of the gas detection device to the gas having the first temperature and is also obtained in step S22, and S1' is a sensitivity of the gas detection device to the gas having the first temperature after the second compensation.

The sensitivity index of the gas detection device to the gas after the second compensation takes into account the influence of environmental temperature changes on sensitivity, and thus the gas detection device is more accurate and is especially suitable for use in scenarios requiring high-precision detection.

Step S26: determine a first relationship between a baseline drift of the gas detection device with respect to a gas concentration and an environmental temperature.

This step corresponds to step S10 in the aforementioned first embodiment.

Step S28: perform first compensation on a gas concentration output value of the gas having the first temperature determined by the gas detection device.

This step can correspond to step S12 in the aforementioned first embodiment.

The inventor has found that in the case that sensitivity compensation (second compensation) is performed first and then baseline drift compensation (first compensation) is performed, a detection result (namely, a gas concentration in the unit of PPM) output by the gas detection device conforms more to the actual index of the gas. Therefore, the aforementioned second embodiment is a more preferred embodiment of the present invention.

A third embodiment of the present invention provides a gas detection device that includes at least a first compensation unit. The first compensation unit is configured to acquire a baseline drift of the gas detection device with respect to a gas having a first temperature, and then perform, based on the baseline drift, a first compensation on a gas concentration output value of the gas having the first temperature determined by the gas detection device. The baseline drift may be acquired by the first compensation unit from the outside of the device or acquired from other units inside the device.

As a specific implementation, when delivered from the factory, the storage unit of the gas detection device already has a first relationship provided by the manufacturer stored thereon. The first relationship indicates a correspondence between a baseline drift of the gas detection device with respect to a gas concentration and an environmental temperature, and the first relationship may be represented as a quadratic functional equation. In practical application scenarios, a calculation unit may directly acquire the first relationship from the storage unit, and accordingly calculate a baseline drift of the gas detection device with respect to a gas having a first temperature. The compensation unit acquires the baseline drift from the calculation unit and performs a first compensation on an obtained detected gas concentration output value of the gas having the first temperature.

As another specific implementation, the first compensation unit acquires a baseline drift of the gas detection device with respect to a gas having a certain temperature from an external independent system or from the cloud so as to directly perform a compensation operation, or acquires the aforementioned first relationship, then calculates a baseline drift, and afterwards performs a compensation operation.

Preferably, the gas detection device further includes a second compensation unit. The second compensation unit is configured to acquire a first sensitivity of the device to a gas having a calibration point temperature and a second sensitivity to the gas having the first temperature, and perform a second compensation on the second sensitivity based on the first sensitivity and an actual sensitivity of the device to the gas having the calibration point temperature. The first and second sensitivities may be provided by a system external to the device. Alternatively, the system external to the device or the cloud provides a second relationship that indicates a relationship between a sensitivity of the gas detection device to a gas and an environmental temperature, and a calculation unit inside the device then calculates the first and second sensitivities according to the second relationship.

According to the improved implementation of the aforementioned third embodiment, the gas detection device 30 further includes a first fitting unit 301. The first fitting unit 301 is configured to determine a first relationship between a baseline drift of the gas detection device 30 with respect to a gas concentration and an environmental temperature. The first compensation unit 311 is coupled to the first fitting unit 301 and performs, based on the baseline drift of the gas detection device 30 with respect to a gas having a first temperature, a first compensation on a gas concentration output value of the gas having the first temperature determined by the gas detection device 30.

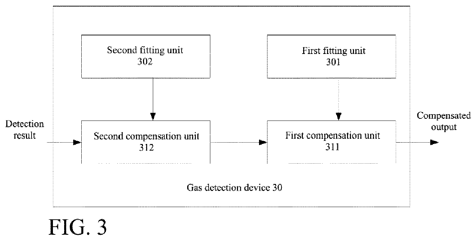

As a further improvement, as shown in FIG. 3, such a gas detection device 30 may further include a second fitting unit 302. The second fitting unit 302 is configured to determine a second relationship between a sensitivity of the gas detection device 30 to a gas and an environmental temperature. The second compensation unit 312 is coupled to the second fitting unit 302 and separately calculates, according to the second relationship, a first sensitivity of the gas detection device 30 to a gas having a calibration point temperature and a second sensitivity to the gas having the first temperature. Alternatively, the first and second sensitivities may be directly calculated and determined by the second fitting unit 302. The second compensation unit 312 further performs a second compensation on the second sensitivity based on the first sensitivity and an actual sensitivity of the gas detection device 30 to the gas having the calibration point temperature.

As shown in FIG. 3, a detection result of the gas detection device 30 is first compensated for the sensitivity index by the second compensation unit 312, then compensated for the baseline value by the first compensation unit 311, and finally presented as a compensated output by the gas detection device 30 on a display interface provided to a user for reading.

In some embodiments of the present invention, the gas detection device 30 further includes a communication unit (not shown in the figure). The communication unit communicates with an external system to acquire fitting information and configures the first relationship and/or second relationship according to the acquired fitting information. As an example, the external system may obtain laboratory data of a baseline drift of the gas detection device 30 with respect to a gas concentration and an environmental temperature, and may further obtain laboratory data of a sensitivity of the gas detection device 30 to a gas and an environmental temperature, and can generate fitting information by performing fitting processing on the data. According to requirements, the communication unit acquires such fitting information from the external system to configure the first relationship and/or second relationship required by the gas detection device 30, so as to implement baseline drift compensation and sensitivity compensation operations.

In some embodiments of the present invention, at least a part of the device may be implemented using a group of distributed computing devices connected to a communication network or implemented based on the "cloud". In such a system, a plurality of computing devices operates together to provide services by using their shared resources.

As an example, the first fitting unit 301 and the second fitting unit 302 are disposed on the cloud, while the first compensation unit 311 and the second compensation unit 312 are disposed on the local end. In other words, the gas detection device 30 is actually implemented as a distributed system. If desired, technicians may invoke the first fitting unit 301 and/or the second fitting unit 302 on the cloud and correspondingly combine the first compensation unit 311 and the second compensation unit 312 on the local end to perform the first and second compensation processes. Preferably, the first fitting unit 301 and the second fitting unit 302 are shared by a plurality of different gas detection devices of the same type so as to achieve full utilization of shared resources.

The implementation based on the "cloud" can have one or a plurality of advantages including: openness, flexibility, expandability, central management, reliability, scalability, optimization for computing resources, and capabilities of aggregating and analyzing information across a plurality of users, connection across a plurality of geographic areas, and applying a plurality of mobile or data network operators to network connectivity.

The foregoing description is intended only for the preferred embodiments of the present invention, but not for limiting the protection scope of the present invention. Those skilled in the art may make various variant designs without departing from the ideas and accompanying claims of the present invention.

**Claims**

1. A method for detecting a gas concentration using a gas detection device, the method comprising:

   a) determining a first relationship between a baseline drift of the gas detection device with respect to a gas concentration and an environmental temperature; and
   b) performing, based on the baseline drift of the gas detection device with respect to a gas having a first temperature, a first compensation on a gas concentration output value of the gas having the first temperature determined by the gas detection device.

2. The method according to claim 1, wherein the first relationship comprises a first functional relationship $Y=F_1(T)$, where T is the environmental temperature and Y is the baseline drift.

3. The method according to claim 2, wherein the first functional relationship is the following: $Y=a_1T^2+b_1T+C_1$, where $a_1$, $b_1$, and $c_1$ are constants determined via a fitting process.

4. The method according to claim 2, wherein the first compensation is calculated according to the following formula:
   $C'=C-Y_1$, where C is an original gas concentration output value of the gas detection device, $Y_1$ is the baseline drift of the gas detection device with respect to the gas having the first temperature, and C' is a gas concentration output value of the gas detection device after the first compensation.

5. The method according to claim 1, wherein the method further comprises:

   determining a second relationship between a sensitivity of the gas detection device to a gas and an environmental temperature;
   separately calculating a first sensitivity of the gas detection device to a gas having a calibration point temperature and a second sensitivity to the gas having the first temperature according to the second relationship; and
   performing a second compensation on the second sensitivity based on the first sensitivity and an actual sensitivity of the gas detection device to the gas having the calibration point temperature.

6. The method according to claim 5, wherein the second relationship comprises a second functional relationship $S=F_2(T)$, where T is the environmental temperature and S is the sensitivity.

7. The method according to claim 6, wherein the second functional relationship is the following: $S=a_2T^2+b_2T+C_2$, where $a_2$, $b_2$, and $c_2$ are constants determined via a fitting process.

8. The method according to claim 5, wherein the second compensation is calculated according to the following formula:

$$S_0'/S_0 = S_1'/S_1,$$

   where $S_0$ is the first sensitivity of the gas detection device to the gas having the calibration point temperature, $S_0'$ is the actual sensitivity of the gas detection device to the gas having the calibration point temperature, S1 is the second sensitivity of the gas detection device to the gas having the first temperature, and $S_1'$ is a sensitivity of the gas detection device to the gas having the first temperature after the second compensation.

9. The method according to any one of claims 5 to 8, wherein before the first compensation is performed, the second compensation is performed.

**10.** A gas detection device, comprising:
a first compensation unit, configured to:

acquire a baseline drift of the gas detection device with respect to a gas having a first temperature; and
perform, based on the baseline drift, a first compensation on a gas concentration output value of the gas having the first temperature determined by the gas detection device.

**11.** The gas detection device according to claim 10, wherein the gas detection device further comprises:
a second compensation unit, configured to:

acquire a first sensitivity of the gas detection device to a gas having a calibration point temperature and a second sensitivity to the gas having the first temperature; and
perform a second compensation on the second sensitivity based on the first sensitivity and an actual sensitivity of the gas detection device to the gas having the calibration point temperature.

**12.** The gas detection device according to claim 10, wherein the gas detection device further comprises:
a first fitting unit, configured to determine a first relationship between a baseline drift of the gas detection device with respect to a gas concentration and an environmental temperature.

**13.** The gas detection device according to claim 11, wherein the gas detection device further comprises:
a second fitting unit, configured to determine a second relationship between a sensitivity of the gas detection device to a gas and an environmental temperature.

**14.** The gas detection device according to any one of claims 10 to 13, wherein the gas detection device further comprises a communication unit, the communication unit communicating with an external system to acquire fitting information to configure the first relationship and/or the second relationship.

**15.** A machine-readable storage medium, having a batch of computer-executable instructions stored thereon, wherein the computer-executable instructions, when executed by a processor, implement the method according to any one of claims 1 to 9.

| Determine a first relationship between a baseline drift of a gas detection device with respect to a gas concentration and an environmental temperature | Step S10 |
|---|---|

| Perform a first compensation on a gas concentration output value of a gas having a first temperature determined by the gas detection device | Step S12 |
|---|---|

## FIG. 1

| Determine a second relationship between a sensitivity of a gas detection device to a gas and an environmental temperature | Step S20 |
|---|---|

| Calculate a first sensitivity of the gas detection device to a gas having a calibration point temperature and a second sensitivity to a gas having a first temperature | Step S22 |
|---|---|

| Perform a second compensation on the second sensitivity | Step S24 |
|---|---|

| Determine a first relationship between a baseline drift of the gas detection device with respect to a gas concentration and an environmental temperature | Step S26 |
|---|---|

| Perform a first compensation on a gas concentration output value of a gas having a first temperature determined by the gas detection device | Step S28 |
|---|---|

## FIG. 2

FIG. 3

Europäisches
Patentamt

European
Patent Office

Office européen
des brevets

## EUROPEAN SEARCH REPORT

Application Number

EP 19 18 7155

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 510 814 A1 (MATSUSHITA ELECTRIC IND CO LTD [JP]) 2 March 2005 (2005-03-02) | 1-15 | INV. G01N33/00 |
| Y | * paragraphs [0008], [0028], [0035] - [0042], [0053]; figure 18 * | 14 | |
| X | GB 2 544 575 A (DRAYSON TECH (EUROPE) LTD [GB]) 24 May 2017 (2017-05-24) | 1,10 | |
| Y | * page 7, lines 19-27 * <br> * page 8, lines 1-25 * <br> * page 11, line 8 - page 12, line 17 * <br> * page 13, lines 1-24 * | 14 | |
| X | EP 1 947 454 A1 (LIFE SAFETY DISTRIBUTION AG [CH]) 23 July 2008 (2008-07-23) <br> * abstract * <br> * paragraphs [0010] - [0014], [0017] - [0033], [0044] - [0050] * | 1,10 | |
| X | US 2013/301052 A1 (MACGREGOR CALUM JOHN [GB] ET AL) 14 November 2013 (2013-11-14) <br> * abstract * <br> * paragraphs [0008], [0013] - [0015], [0017] - [0021], [0024] - [0027], [0029], [0048] - [0051] * | 1,10 | |

TECHNICAL FIELDS
SEARCHED (IPC)

G01N

The present search report has been drawn up for all claims

| Place of search | Date of completion of the search | Examiner |
|---|---|---|
| Munich | 13 December 2019 | Knoll, Stephan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 18 7155

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2019

| Patent document cited in search report | | | Publication date | Patent family member(s) | | | Publication date |
|---|---|---|---|---|---|---|---|
| EP 1510814 | A1 | | 02-03-2005 | EP<br>JP<br>JP<br>US<br>WO | 1510814<br>4165300<br>2004354210<br>2005228596<br>2004106909 | A1<br>B2<br>A<br>A1<br>A1 | 02-03-2005<br>15-10-2008<br>16-12-2004<br>13-10-2005<br>09-12-2004 |
| GB 2544575 | A | | 24-05-2017 | AU<br>CA<br>CN<br>EP<br>GB<br>GB<br>JP<br>US<br>WO | 2017289711<br>3029383<br>109952493<br>3475664<br>2544575<br>2545137<br>2019527344<br>2019234920<br>2018002602 | A1<br>A1<br>A<br>A1<br>A<br>A<br>A<br>A1<br>A1 | 31-01-2019<br>04-01-2018<br>28-06-2019<br>01-05-2019<br>24-05-2017<br>07-06-2017<br>26-09-2019<br>01-08-2019<br>04-01-2018 |
| EP 1947454 | A1 | | 23-07-2008 | CA<br>EP<br>US | 2617856<br>1947454<br>2008173065 | A1<br>A1<br>A1 | 22-07-2008<br>23-07-2008<br>24-07-2008 |
| US 2013301052 | A1 | | 14-11-2013 | CA<br>CN<br>EP<br>JP<br>JP<br>US<br>WO | 2853962<br>103328954<br>2635893<br>5959524<br>2013541019<br>2013301052<br>2012059743 | A1<br>A<br>A2<br>B2<br>A<br>A1<br>A2 | 10-05-2013<br>25-09-2013<br>11-09-2013<br>02-08-2016<br>07-11-2013<br>14-11-2013<br>10-05-2012 |

EPO FORM P0459

For more details about this annex : see Official Journal of the European Patent Office, No. 12/82